# EUROPÄISCHE PATENTANMELDUNG

(11) **EP 3 569 594 A1**
(43) Veröffentlichungstag der Anmeldung: **20.11.2019**
(21) Anmeldenummer: 18172926.0
(22) Anmeldetag: 17.05.2018
(51) Int. Cl.: C07D 241/24

(54) **VERFAHREN ZUR HERSTELLUNG VON SUBSTITUIERTEN PYRAZINEN**

(71) Anmelder: Universität Graz, 8010 Graz (AT); ACIB GmbH, 8010 Graz (AT)
(72) Erfinder: KROUTIL, Wolfgang, 8042 Graz (AT); VELIKOGNE, Stefan, 8010 Graz (AT)
(74) Vertreter: Ellmeyer, Wolfgang

(57) **Zusammenfassung**

Die Erfindung betrifft ein Verfahren zur Herstellung von substituierten Pyrazinen ausgehend von aktivierten Ketoximen mittels enzymatischer Katalyse, wobei ein durch die nachstehende allgemeine Formel (1) dargestelltes Derivat der 2-Oximinoacetessigsäure in Gegenwart eines Enzyms aus der Klasse der En-Reduktasen (ER) und eines Hydridionen übertragenden Coenzyms wie NADH/H⁺ oder NADPH/H⁺ (NAD(P)H/H⁺) in wässriger Pufferlösung unter Dimerisierung, Ringschluss und Aromatisierung zum 3,6-disubstituierten Pyrazin-2,5-dicarbonsäure-Derivat (2) umgesetzt wird: 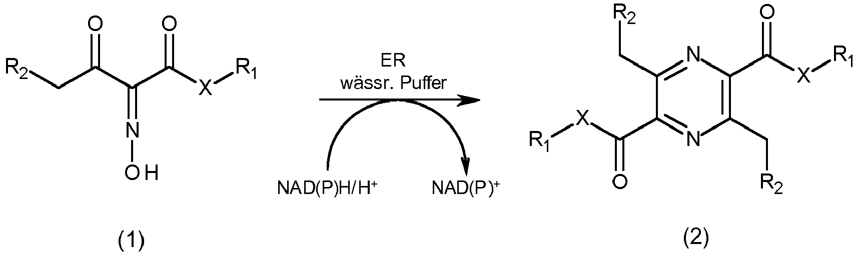 worin
X aus O, S, NH und NR₁ ausgewählt ist;
R₁ jeweils unabhängig voneinander aus linearen oder verzweigten, gesättigten oder ungesättigten aliphatischen und aromatischen Kohlenwasserstoffen mit 1 bis 10 Kohlenstoffatomen ausgewählt ist, in denen ein oder mehrere Kohlenstoffatome gegebenenfalls durch ein aus Sauerstoff, Stickstoff und Schwefel ausgewähltes Heteroatom ersetzt sind; und
R₂ aus Wasserstoff und den Optionen für R₁ ausgewählt ist.

## Beschreibung

Die vorliegende Erfindung betrifft ein Verfahren zur Herstellung von substituierten Pyrazinen ausgehend von aktivierten Ketoximen mittels enzymatischer Katalyse.

### STAND DER TECHNIK

Pyrazine sind wichtige heterozyklische aromatische Verbindungen, die in der Natur nahezu ubiquitär vorkommen. Sie sind sehr begehrte Moleküle, da von einigen berichtet wurde, dass sie vorteilhafte physiologische Aktivitäten zeigen, wie z.B. 2,3,5,6-Tetramethylpyrazin, Coelenterazin und Coelenteramin sowie Amilorid. Neben ihrer Anwendung im medizinischen Bereich werden Alkylpyrazine aufgrund ihrer gerösteten und nussigen Geruchsnoten, die auch zum typischen Geschmack und Aroma von Kaffee beitragen, häufig in der Aroma- und Duftstoffindustrie eingesetzt. Zusätzlich wurde bei Orchideen, die eine sexuelle Mimikry entwickelt haben, festgestellt, dass sie ein Gemisch aus Alkyl- und Hydroxymethylpyrazinen als Botenstoffe produzieren, um bestäubende Insekten anzulocken.

In neueren Untersuchungen wurden 3,6-Dimethylpyrazin-2,5-dicarbonsäureester als potentielle Aromastoffvorläufer für die Aromatisierung von bei hohen Temperaturen zu verarbeitenden Gütern identifiziert. Bei der Pyrolyse können diese Verbindungen einen geruchsaktiven Alkohol und ein alkyliertes Pyrazin freisetzen, die zum Aroma von beispielsweise Tabak beitragen (Lai et al., J. Therm. Anal. Calorim. 123, 479-487 (2015); Lai et al., J. Therm. Anal. Calorim. 128, 1627-1638 (2016)).

Zur Herstellung solcher Verbindungen wurden im Laufe der Jahre mehrere Verfahren beschrieben, allerdings umfassen viele dieser Verfahren raue Reaktionsbedingungen, Schwer- oder Edelmetallkatalysatoren, lange Reaktionszeiten und nicht leicht verfügbare Ausgangsprodukte (Palacios et al., Org. Lett. 4, 2405-2408 (2002); Gnanaprakasam et al., Angew. Chem. Int. Ed. 50,12240-12244 (2011); Viswanadham et al., Chem. Commun. 50, 13517-13520 (2014); Ryu et al., J. Org. Chem. 80, 2376-2383 (2015); Pandit et al., Adv. Synth. Catal. 358, 3586-3599 (2016); Reiser et al., Synlett. 28, 1707-1714 (2018)).

Um diese Nachteile zu umgehen, stellt die Biokatalyse eine geeignete Alternative für die Herstellung solcher Verbindungen dar. Die Reaktionsbedingungen für biokatalytische Umsetzungen umfassen üblicherweise (gepufferte) wässrige Lösungen, Umgebungstemperaturen und relativ billige und leicht verfügbare Katalysatoren, die in großen Mengen von Mikroorganismen produziert werden können. Darüber hinaus wird eine hohe Selektivität für die umgesetzte funktionelle Gruppe üblicherweise durch den Einsatz von Enzymen oder Ganzzell-Biokatalysatoren erreicht. Literaturberichte über enzymatische oder mikrobielle Pyrazinproduktion sind jedoch selten, und die verantwortlichen Enzyme wurden nur in wenigen Fällen identifiziert (Kwang-Soo et al., J. Microbiol. Biotechnol. 4, 327-332 (1994); Besson et al., Appl. Microbiol. Biotechnol. 47, 489-495 (1997); Payer et al., Eur. J. Org. Chem. 2017(17), 2553-2559 (2017)). Mo et al., J. Mol. Catal. B: Enzym. 123, 29-34 (2016), haben herausgefunden, dass Backhefe-Ganzzellpräparate ausgehend von β-Keto-α-oximinoestern und -amiden die entsprechenden 3,6-Dimethylpyrazin-2,5-dicarbonsäure-Derivate produzierten. Welche Enzyme genau an dieser Reaktion beteiligt waren, wurde allerdings nicht aufgeklärt. Die Reaktionen werden als stark vom pH-Wert des Reaktionsmediums abhängig beschrieben, wobei das Optimum bei pH 5 festgestellt wurde.

Ziel der Erfindung war vor diesem Hintergrund, ein verbessertes, insbesondere besser reproduzierbares, enzymatisch katalysiertes Verfahren zur Herstellung von Pyrazindicarbonsäure-Derivaten zu entwickeln.

### ZUSAMMENFASSUNG DER ERFINDUNG

Dieses Ziel erreicht die vorliegende Erfindung durch Bereitstellung eines Verfahrens zur Herstellung von substituierten Pyrazinen ausgehend von aktivierten Ketoximen mittels enzymatischer Katalyse, wobei ein durch die nachstehende allgemeine Formel (1) dargestelltes Derivat der 2-Oximinoacetessigsäure in Gegenwart eines Enzyms aus der Klasse der En-Reduktasen (ER) und eines Hydridionen übertragenden Coenzyms wie NADH/H⁺ oder NADPH/H⁺ (NAD(P)H/H⁺) in wässriger Pufferlösung unter Dimerisierung, Ringschluss und Aromatisierung zum 3,6-disubstituierten Pyrazin-2,5-dicarbonsäure-Derivat (2) umgesetzt wird: 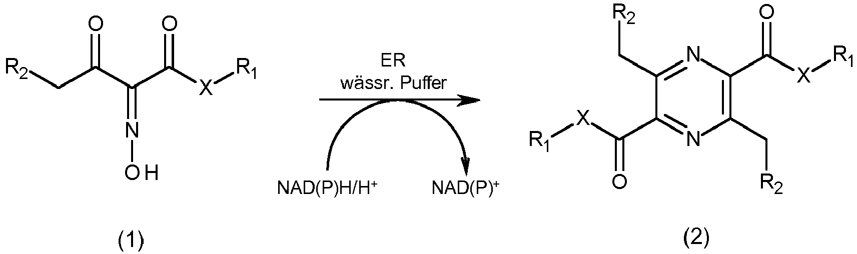 worin
X aus O, S, NH und NR₁ ausgewählt ist;
R₁ jeweils unabhängig voneinander aus linearen oder verzweigten, gesättigten oder ungesättigten aliphatischen und aromatischen Kohlenwasserstoffen mit 1 bis 10 Kohlenstoffatomen ausgewählt ist, in denen ein oder mehrere Kohlenstoffatome gegebenenfalls durch ein aus Sauerstoff, Stickstoff und Schwefel ausgewähltes Heteroatom ersetzt sind; und
R₂ aus Wasserstoff und den Optionen für R₁ ausgewählt ist.

Die Erfinder haben nämlich völlig überraschenderweise festgestellt, dass für die Umsetzung von β-Keto-α-oximinoestern und -amiden zu 2,5-Pyrazindicarbonsäure-Derivaten durch Ganzzell-Präparate verschiedener Mikroorganismen nicht etwa Imin-Reduktasen, wie dies der Fachmann vermutet hätte, sondern En-Reduktasen (ER) verantwortlich sind. Diese sind eigentlich nur für die selektive Katalyse der Reduktion aktivierter C-C-Doppelbindungen, wie z.B. von α,β-ungesättigten Aldehyde, Ketonen oder Carbonsäuren und deren Derivaten, nicht aber jener von Iminen oder Oximen bekannt. Nichtsdestotrotz sind En-Reduktasen offenbar in der Lage, Oxime zu Aminen oder Hydroxylaminen zu reduzieren, die in der Folge zu nukleophilen Angriffen an Carbonylgruppen in der Lage sind. Diese Feststellung der Erfinder ermöglicht die Durchführung der obigen Synthesen von 2,5-Pyrazindicarbonsäure-Derivaten aus entsprechenden 2-Oximinoacetessigsäure-Derivaten auf wesentlich besser reproduzierbare Weise unter Verwendung einer jeweils am besten für die Reaktion geeigneten ER als Enzymkatalysator. Zumal zwar alle getesteten ER praktisch vollständigen Umsatz des jeweiligen Substrats, d.h. 2-Oximinoacetessigsäure-Derivats, herbeiführten - und das trotz des Einsatzes verschiedenster Reste R₁ und R₂ -, aber die Ausbeuten der gewünschten Pyrazin-Derivate starken Schwankungen unterlagen, wie die späteren Beispiele belegen.

So haben die Erfinder herausgefunden, dass speziell für den Ester- oder Amid-Substituenten R₁ ein großer Spielraum besteht, indem sie verschiedene gesättigte, ungesättigte und aromatische Reste, mitunter sogar mit Heteroatomen in der Struktur, untersuchten und in allen Fällen praktisch vollständigen Umsatz feststellen konnten.

In bevorzugten Ausführungsformen ist R₁ jedoch kein allzu langer oder voluminöser Rest, sondern ist aus C₁₋₆-Alkyl, C₁₋₆-Alkenyl, Phenyl und Benzyl ausgewählt, worin ein oder mehrere Kohlenstoffatome gegebenenfalls durch Sauerstoff ersetzt sind. Allerdings ist mit hoher Wahrscheinlichkeit anzunehmen, dass ein Schwefel- oder Stickstoffatom anstelle des Sauerstoffatoms innerhalb der Struktur von R₁ die Reaktion nicht negativ beeinflussen wird, weswegen diese ebenso im Schutzumfang der vorliegenden Erfindung liegen sollen wie Thioester, d.h. Derivate, in denen X S ist, wenngleich aus Gründen der Verfügbarkeit der Ausgangsprodukte X vorzugsweise aus O und NH ausgewählt ist. Wenn X NR₁ ist, d.h. das gewünschte Derivat ein N,N-disubstituiertes Amid ist, sollte R₁ ein relativ kleiner Rest, vorzugsweise ein C₁₋₃-Alkylrest, sein.

Der ω-Substituent R₂ des 2-Oximinoacetessigsäure-Ausgangsprodukts ist vorzugsweise aus Wasserstoff und C₁₋₆-Alkyl, insbesondere aus Wasserstoff und Methyl, ausgewählt, um keine sterische Hinderung für den enzymatischen Angriff am Oxim darzustellen.

Die En-Reduktase ist zwar nicht speziell eingeschränkt, ist aber vorzugsweise aus der OYE-Familie, d.h. der Familie der "Old Yellow Enzymes" ausgewählt, da Vertreter dieser Gruppe von Enzymen allesamt gute Ergebnisse geliefert haben. Noch bevorzugter ist die ER aus den folgenden bereits getesteten Enzymen ausgewählt:
OYE1 von *Saccharomyces pastorianus,*
OYE2 von *Saccharomyces cerevisiae,*
OYE3 von *Saccharomyces cerevisiae,*
12-Oxophytodienoat-Reduktase 3 (OPR3) von *Arabidopsis thaliana,*
xenobiotischer Reduktase A (XenA) von *Pseudomonas putida* II-B und
En-Reduktase von *Ferrovum* sp. JA12 (FOYE).

Das Enzym kann dabei isoliert, d.h. in Reinform, aber auch in Form von die ER exprimierenden Ganzzellen, mitunter in lyophilisierter Form, eingesetzt werden.

Die wässrige Pufferlösung weist vorzugsweise einen pH von 7 bis 8, noch bevorzugter einen pH von etwa 7,5, also einen schwach alkalischen pH-Wert auf, da in diesem pH-Bereich praktisch vollständige Umsätze beobachtet wurden. Dieser Umstand ist überraschend, da Mo et al. (s.o.) ein pH-Optimum im schwach sauren Bereich, nämlich einen pH von 5 für die ihre mit Backhefe-Ganzzellpräparaten katalysierten Reaktionen festgestellt hatten. Der durchschnittliche Fachmann ist jedoch ohne Weiteres in der Lage, das pH-Optimum für die jeweils eingesetzte ER durch einfache Routineversuche zu bestimmen.

Dasselbe gilt für die optimale Reaktionstemperatur, die ebenfalls nicht speziell eingeschränkt ist. Vorzugsweise wird die Reaktion im erfindungsgemäßen Verfahren jedoch bei den für Biokatalysen üblichen Temperaturen im Bereich von 20 °C bis 35 °C, noch bevorzugter bei etwa 30 °C, durchgeführt.

In besonders bevorzugten Ausführungsformen der vorliegenden Erfindung wird das Coenzym unter Verwendung einer Dehydrogenase samt zugehörigem Substrat rezykliert, da die Erfinder festgestellt haben, dass sich dadurch die Reaktionszeiten bis zur Erzielung vollständiger Umsätze deutlich verkürzen lassen. In bevorzugten Ausführungsformen wird zur Rezyklierung des Coenzyms eine Kombination aus Glucose und Glucose-Dehydrogenase (GDH) oder aus Phosphit und Phosphit-Dehydrogenase (PDH) eingesetzt, insbesondere eine Kombination aus Glucose und GDH, die die Reaktionszeit gegenüber PDH noch weiter verkürzt.

### KURZBESCHREIBUNG DER ZEICHNUNG

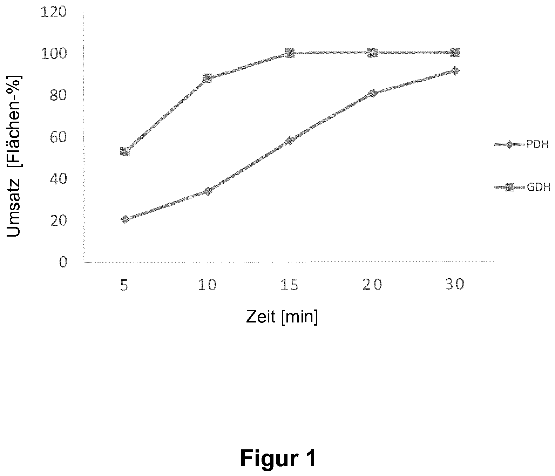

In der einzigen Zeichnung, Fig. 1, ist ein Vergleich der unterschiedlichen Wirkung von GDH und PDH auf die Reaktionszeit bei der Durchführung des erfindungsgemäßen Verfahrens grafisch dargestellt.

### BEISPIELE

Die vorliegende Erfindung wird nachstehend anhand von illustrativen Beispielen näher beschrieben, die jedoch lediglich als exemplarisch und nicht als Einschränkung des ausschließlich in den beiliegenden Ansprüchen definierten Schutzbereichs anzusehen sind.

Alle Ausgangsprodukte und Reagenzien wurden aus kommerziellen Quellen bezogen und ohne weitere Reinigung direkt eingesetzt, sofern nichts anderes angegeben ist. Die chemischen Bezeichnungen und Abkürzungen haben die üblichen Bedeutungen, die durchschnittlichen Fachleuten allgemein bekannt (oder hierin beschrieben) sind.

### Synthesebeispiele 1 bis 6

### Heterologe Expression von sechs En-Reduktasen

Plasmide, die jeweils ein für eine der En-Reduktasen OYE1, OYE2, OYE3, OPR3, XenA und FOYE kodierendes Gen enthielten, wurden in chemisch kompetente *E. coli* BL21(DE3)-Zellen transformiert. Die sechs Über-Nacht-Kulturen wurden in LB-Medium, das mit 50 µg/ml Kanamycin (für OYE1-3 und FOYE) oder 100 µg/ml Ampicillin (für OPR3 und XenA) ergänzt worden war, bei 30 °C und 120 U/min kultiviert. Hauptkulturen (1 l LB-Medium, 50 µg/ml Kanamycin oder 100 µg/ml Ampicillin) wurden mit je 3 ml der entsprechenden Über-Nacht-Kultur inokuliert und bei 37 °C, 120 U/min geschüttelt, bis die OD₆₀₀ 0,6 bis 0,8 erreichte. Die Proteinproduktion wurde durch Zusatz von Isopropyl-*β*-D-thiogalactopyranosid (IPTG) (0,2 mM für OYE1-3 und XenA, 1 mM für OPR3 und 0,05 mM für FOYE) induziert und bei 20 °C über Nacht (für OYE1-3 und FOYE) oder bei 37 °C 4 h lang (für XenA und OPR3) geschüttelt. Die Zellen wurden durch Zentrifugation (5000 U/min, 4 °C, 20 min) geerntet, das Pellet wurde mit 100 mM Tris-HCI-Puffer pH 7,5 (25 ml) gewaschen und bei -20 °C gelagert.

### Synthesebeispiele 7 bis 13

### Herstellung von 2-Oximinoacetessigsäure-Derivaten (1)

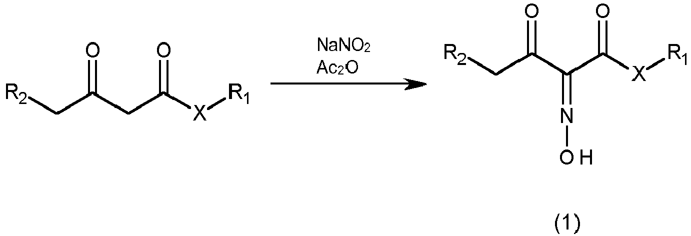

### Allgemeine Vorgangsweise:

Zu einer gerührten Lösung des entsprechenden Acetessigsäureesters oder -amids (1,0 Äquiv.) in Eisessig (10-15 ml) wurde eine Lösung von NaNO₂ (1,5 Äquiv.) in Wasser bei 0 ° C zugetropft. Nach vollständiger Zugabe wurde das Reaktionsgemisch auf Raumtemperatur erwärmen gelassen und gerührt, bis mittels DC vollständiger Umsatz festgestellt wurde. Die Reaktion wurde mit festem Na₂CO₃ neutralisiert, die wässrige Phase wurde mit EtOAc (3 × 20 ml) extrahiert, und die vereinigten organischen Phasen wurden über Na₂SO₄ getrocknet. Das Lösungsmittel wurde unter reduziertem Druck abdestilliert, um das jeweilige rohe Oximprodukt (1) zu erhalten.

### Svnthesebeispiel 7: Herstellung von 2-Oximinoacetessigsäure-N-phenylamid (1b)

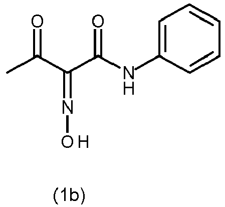

Das gemäß der obigen allgemeinen Vorgangsweise aus Acetessigsäure-N-phenylamid (oder: -anilid) erhaltene rohe Oxim wurde mittels Flash-Chromatographie (EtOAc:Cyclohexan 1:2, Rf = 0,40) gereinigt, um Oxim (1b) in Form von gelben Kristallen zu erhalten (Ausbeute: 57 % d. Th.).
¹H-NMR (300 MHz, CDCl₃) δ = 11,00 (s, 1H), 7,76-7,13 (m, 5H), 2,62 (d, *J* = 10,3 Hz, 3H). ¹³C-NMR (75 MHz, CDCl₃): δ = 200,1, 161,3, 143,5, 135,3, 129,3, 126,3, 121,4, 26,4.

### Synthesebeispiel 8: Herstellung von 2-Oximinoacetessigsäureallylester (1c)

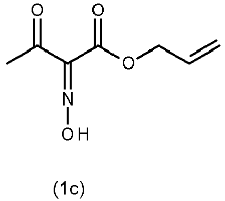

Das gemäß der obigen allgemeinen Vorgangsweise aus Acetessigsäureallylester erhaltene rohe Oxim wurde mittels Flash-Chromatographie (EtOAc:Cyclohexan 1:4, R_{f} = 0,23) gereinigt, um Oxim (1c) als gelbes Öl zu erhalten (Ausbeute: 20 % d. Th.).
¹H-NMR (300 MHz, CDCl₃) δ = 9,46 (s, 1H), 5,94 (m, 1H), 5,35 (m, 2H), 4,81 (dt, 2H), 2,45 (d, 3H). ¹³C-NMR (75 MHz, CDCl₃): δ = 193,9, 161,4, 151,1,130,7, 120,1, 119,6, 66,8, 25,5.

### Synthesebeispiel 9: Herstellung von 2-Oximinoacetessigsäurebutylester (1d)

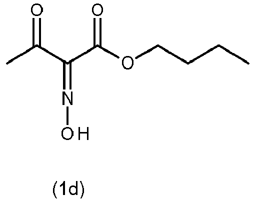

Das gemäß der obigen allgemeinen Vorgangsweise aus Acetessigsäurebutylester erhaltene rohe Oxim wurde mittels Flash-Chromatographie (EtOAc:Cyclohexan 1:4, R_{f} = 0,23) gereinigt, um Oxim (1d) als gelbes Öl zu erhalten (Ausbeute: 29 % d. Th.).
¹H-NMR (300 MHz, CDCl₃) δ = 9,57 (s, 1H), 4,32 (t, 2H), 2,40 (s, 3H), 1,74-1,62 (m, 2H), 1,47-1,33 (m, 2H), 0,93 (t, 3H). ¹³C-NMR(75 MHz, CDCl₃): δ = 193,9, 161,9, 151,3, 66,4, 30,5, 25,5, 19,0, 13,7.

### Synthesebeispiel 10: Herstellung von 2-Oximinoacetessigsäurebenzylester (1e)

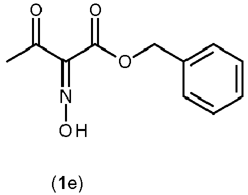

Das gemäß der obigen allgemeinen Vorgangsweise aus Acetessigsäurebenzylester erhaltene rohe Oxim wurde mittels Flash-Chromatographie (EtOAc:Hexan 1:4, Rf = 0,28) gereinigt, um Oxim (1e) als gelbes Öl zu erhalten, das sich bei der Lagerung bei 4 °C verfestigte (Ausbeute: 80 % d. Th.).
Fp.: 78-79 °C (Lit.: 81-82 °C; Doyle et al., Can. J. Chem. 55, 484-507 (1977))
¹H-NMR (300 MHz, CDCl₃) δ = 9,26 (s, 1H), 7,37 (m, 5H), 5,35 (s, 2H), 2,43 (d, 3H). ¹³C-NMR (75 MHz, CDCl₃): δ = 193,8, 161,9, 151,3, 134,6, 128,8, 128,5, 67,8, 25,4.

### Synthesebeispiel 11: Herstellung von 2-Oximinoacetessigsäureheptylester (1f)

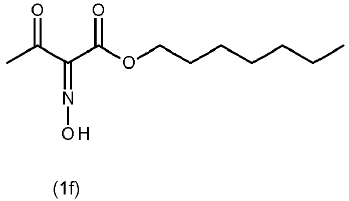

Das gemäß der obigen allgemeinen Vorgangsweise aus Acetessigsäureheptylester erhaltene rohe Oxim wurde mittels Flash-Chromatographie (EtOAc:Hexan 1:4, Rf = 0,28) gereinigt, um Oxim (1f) als gelbes Öl zu erhalten (Ausbeute: 92 % d. Th.).
¹H-NMR (300 MHz, CDCl₃) δ = 9,43 (s, 1H), 4,31 (t, 2H), 2,41 (s, 3H), 1,80-1,60 (m, 2H), 1,45-1,17 (m, 8H), 0,88 (dd, 3H). ¹³C-NMR (75 MHz, CDCl₃): δ = 193,8, 161,9, 151,3, 66,7, 31,8, 28,9, 28,5, 25,7, 25,6, 22,7, 14,2.

### Synthesebeispiel 12: Herstellung von 2-Oximinoacetessigsäure-2-methoxyethylester (1g)

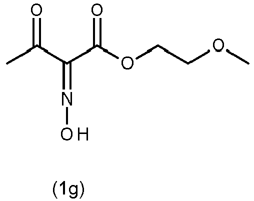

Das gemäß der obigen allgemeinen Vorgangsweise aus Acetessigsäure-2-methoxy-ethylester (oder -ethylenglykolester) erhaltene rohe Oxim wurde mittels Flash-Chro-matographie (CHCl₃:MeOH 95:5, R_{f} = 0,42) gereinigt, um Oxim (1g) als farbloses Öl zu erhalten (Ausbeute: 54 % d. Th.).
¹H-NMR (300 MHz, CDCl₃) δ = 4,49-4,44 (m, 2H), 3,77-3,72 (m, 2H), 3,46 (s, 3H), 2,38 (s, 3H). ¹³C-NMR (75 MHz, CDCl₃): δ = 193,8, 161,9, 150,8, 70,3, 64,6, 59,3.

### Synthesebeispiel 13: Herstellung von 4-Methyl-2-oximinoacetessigsäureethylester (1h)

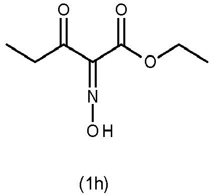

Das gemäß der obigen allgemeinen Vorgangsweise aus Acetessigsäure-2-methoxy-ethylester als gelbes Öl erhaltene rohe Oxim (Ausbeute: 94 % d. Th.) wurde ohne weitere Reinigung in den späteren Reaktionen eingesetzt.
¹H-NMR (300 MHz, CDCl₃) δ = 9,36 (s, 1H), 4,36 (dq, *J* = 12,0, 7,1 Hz, 2H), 2,89-2,69 (m, 2H), 1,34 (td, *J* = 7,1, 3,3 Hz, 3H), 1,14 (dt, J = 13,0, 7,3 Hz, 3H). ¹³C-NMR (75 MHz, CDCl₃): δ = 196,5, 161,8, 150,5, 62,7, 31,2, 14,0, 7,44.

### Beispiele 1 bis 6

### Herstellung von 3,6-Dimethylpyrazin-2,5-dicarbonsäurediethylester (2a)

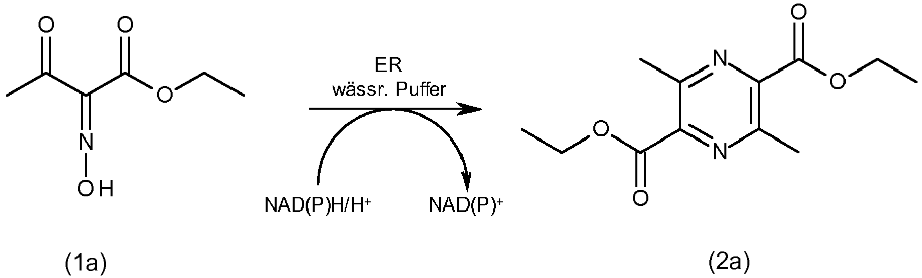

Als Modellreaktion für die Feststellung der Enzymaktivität wurde im Handel erhältlicher 2-Oximinoacetessigsäureethylester (1a) mit den sechs in den Synthesebeispielen 1 bis 6 hergestellten En-Reduktasen (ER) in analytischem Maßstab umgesetzt. Dazu wurden in ein 2-ml-Eppendorf-Röhrchen 100 µg/ml der jeweiligen gereinigten En-Reduktase, 0,5 mM NAD(P)H/H⁺, 50 mM Glucose und 1 mg/ml GDH in 100 mM Tris-HCl-Puffer, pH 7,5, gelöst. Die Reaktionen wurden durch Zugabe von 10 mM Oxim (1a) (aus einer 200 mM Stammlösung in DMSO) auf ein Endvolumen von 500 µl gestartet und 24 h lang bei 30 °C, 120 U/min geschüttelt. Danach wurden die Reaktionsgemische jeweils mit 1 ml Chloroform extrahiert, die organische Phase wurde über MgSO₄ getrocknet und mittels GC-FID analysiert und der Umsatz anhand der Peakflächenprozentsätze ermittelt. Wie aus nachstehender Tabelle 1 hervorgeht, wurde nach 24 h in allen sechs Fällen praktisch vollständiger Umsatz festgestellt.

**Tabelle 1: Anfängliches Reaktivitätsscreening mit Modelloxim (1a)**

| **Beispiel** | **Enzym** | **Oxim** | **Umsatz [%]** |
|---|---|---|---|
| 1 | OYE1 | (1a) | > 99 |
| 2 | OYE2 | (1a) | > 99 |
| 3 | OYE3 | (1a) | > 99 |
| 4 | XenA | (1a) | > 99 |
| 5 | OPR3 | (1a) | > 99 |
| 6 | FOYE | (1a) | > 99 |

### Beispiele 7 und 8

Um zu ermitteln, in welcher Zeit vollständiger Umsatz des Oxims zum Pyrazindicarbonsäureester erzielbar ist, wurde die obige Reaktion unter Verwendung von FOYE als ER in zwei Ansätzen wiederholt, wobei im ersten Fall (Beispiel 7) Phosphit/PDH und im zweiten Fall (Beispiel 8) Glucose/GDH zur Rezyklierung des Coenzyms eingesetzt wurden. Während der Reaktionen wurden alle 5 min Proben gezogen und erneut mittels GC-FID analysiert. Die Ergebnisse sind in Fig. 1 dargestellt.

Aus Fig. 1 ist ersichtlich, dass in Beispiel 7 mit Phosphit/PDH nach 30 min etwa 90 % Umsatz zu beobachten und 100% für eine Reaktionszeit von etwa 45 min zu erwarten waren, während unter Verwendung von Glucose/GDH bereits nach 15 min praktisch vollständiger Umsatz erzielt war. Das System Glucose/GDH ist somit gemäß vorliegender Erfindung zu bevorzugen.

### Beispiele 9 bis 50

Zur Ermittlung, welche anderen Derivate der 2-Oximinoacetessigsäure mittels enzymatischer Katalyse durch die sechs in den Synthesebeispielen 1 bis 6 hergestellten En-Reduktasen (1) zu Pyrazindicarbonsäure-Derivaten (2) umsetzbar sein würden, wurden die für die Beispiele 1 bis 6 beschriebene Vorgangsweise unter Verwendung der in den Synthesebeispielen 7 bis 13 hergestellten Oxime (1b) bis (1h) anstelle von (1a) wiederholt. Überraschenderweise wurde in sämtlichen Fällen nach 24 h praktisch vollständiger Umsatz beobachtet, wie aus nachstehender Tabelle 2 hervorgeht.

**Tabelle 2: Reaktivitätsscreening mit den Oximen (1b) bis (1h)**

| **Beispiel** | **Enzym** | **Oxim** | **Umsatz [%]** |
|---|---|---|---|
| 9 | OYE1 | (1b) | > 99 |
| 10 | OYE2 | (1b) | > 99 |
| 11 | OYE3 | (1b) | > 99 |
| 12 | XenA | (1b) | > 99 |
| 13 | OPR3 | (1b) | > 99 |
| 14 | FOYE | (1b) | > 99 |
| 15 | OYE1 | (1c) | > 99 |
| 16 | OYE2 | (1c) | > 99 |
| 17 | OYE3 | (1c) | > 99 |
| 18 | XenA | (1c) | > 99 |
| 19 | OPR3 | (1c) | > 99 |
| 20 | FOYE | (1c) | > 99 |
| 21 | OYE1 | (1d) | > 99 |
| 22 | OYE2 | (1d) | > 99 |
| 23 | OYE3 | (1d) | > 99 |
| 24 | XenA | (1d) | > 99 |
| 25 | OPR3 | (1d) | > 99 |
| 26 | FOYE | (1d) | > 99 |
| 27 | OYE1 | (1e) | > 99 |
| 28 | OYE2 | (1e) | > 99 |
| 29 | OYE3 | (1e) | > 99 |
| 30 | XenA | (1e) | > 99 |
| 31 | OPR3 | (1e) | > 99 |
| 32 | FOYE | (1e) | > 99 |
| 33 | OYE1 | (1f) | > 99 |
| 34 | OYE2 | (1f) | > 99 |
| 35 | OYE3 | (1f) | > 99 |
| 36 | XenA | (1f) | > 99 |
| 37 | OPR3 | (1f) | > 99 |
| 38 | FOYE | (1f) | > 99 |
| 39 | OYE1 | (1g) | > 99 |
| 40 | OYE2 | (1g) | > 99 |
| 41 | OYE3 | (1g) | > 99 |
| 42 | XenA | (1g) | > 99 |
| 43 | OPR3 | (1g) | > 99 |
| 44 | FOYE | (1g) | > 99 |
| 45 | OYE1 | (1h) | > 99 |
| 46 | OYE2 | (1h) | > 99 |
| 47 | OYE3 | (1h) | > 99 |
| 48 | XenA | (1h) | > 99 |
| 49 | OPR3 | (1h) | > 99 |
| 50 | FOYE | (1h) | > 99 |

Somit scheint die Natur des Carbonsäurederivats für die Aktivität der Enzyme eine relativ untergeordnete Rolle zu spielen, was darauf schließen lässt, dass das Oxim mit dem Keton-Ende voran in das aktive Zentrum des Enzyms eintritt. Aus diesem Grund ist zu vermuten, dass längere Reste R₂ an diesem Ende als der hierin - als Alternative zu Wasserstoff - getestete CH₃-Rest von Oxim (1h), d.h. Reste R₂ mit einer größeren Anzahl an Kohlenstoffatomen, wie z.B. mit mehr als 6 oder mitunter auch schon mit mehr als 3 Kohlenstoffatomen, den Umsatz (deutlich) verringern werden. Allerdings ist zu vermuten, dass auch ein längerkettiger Rest R₁ mit mehr als 10 Kohlenstoffatomen oder ein sperriger Rest R₁, wie z.B. substituiertes Naphthyl oder dergleichen, den Angriff des Enzyms am Oxim-Substrat (1) behindern wird, weswegen gemäß vorliegender Erfindung eine Obergrenze von maximal 10 Kohlenstoffatomen für die Substituenten R₁ und R₂ definiert wird. Näher Untersuchungen der tatsächlichen Grenzen sind derzeit Gegenstand weiterer Experimente der Erfinder.

Überraschenderweise hatte jedoch weder die Gegenwart der relativ sperrigen Reste Phenyl und Benzyl in den Oximen (1b) und (1e), noch die einer NH-Gruppe im Anilid (1b), nach die einer Doppelbindung oder die eines Sauerstoffatoms in den Estergruppierungen der Oxime (1c) und (1g) einen nennenswerten Einfluss auf die Reaktivität der Enzyme: In allen Fällen konnte mittels GC-FID praktisch vollständiger Umsatz zu den jeweiligen Pyrazindicarbonsäure-Derivaten (2a) bis (2h) nachgewiesen werden.

Wie lange im Einzelfall die Reaktionsdauer bis zur vollständigen Umsetzung tatsächlich beträgt, d.h. um wie viel länger die Reaktionen der Oxime (1b) bis (1h) zu den Pyrazinen (2b) bis (2h) als jene von (1a) zu (2a) dauern, was für den Fachmann anzunehmen ist, wird von den Erfindern ebenfalls derzeit untersucht.

### Vergleichsbeispiele 1 bis 3

### Versuch der Herstellung von substituierten 2,5-Diacyl-Pyrazinen (4)

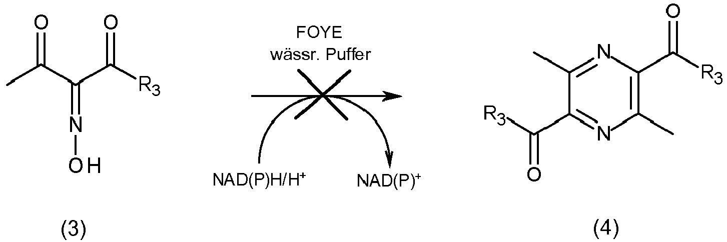

(3a) R₃ = CH₃
(3b) R₃ = Ph
(3c) R₃ = iBu

Zur Untersuchung, ob die enzymatische Katalyse auch mit 2-Oximino-1,3-diketonen anstelle der 2-Oximinoacetessigsäure-Derivate als Substrate abläuft, d.h. ob das Carbonsäure-Ende des Substratmoleküls für die Reaktion erforderlich ist, wurden die drei nachstehend dargestellten Diketon-Oxime (3a), (3b) und (3c) 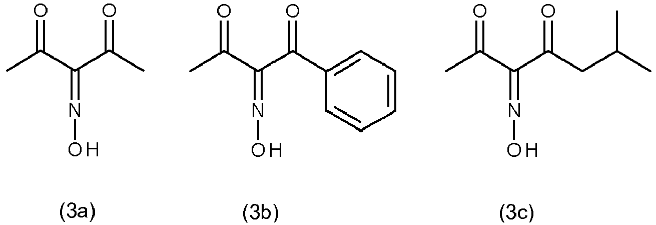 auf analoge Weise zu den obigen Synthesebeispielen 7 bis 13 aus den entsprechenden Diketon-Ausgangsprodukten (Acetylaceton, ω-Acetylacetophenon und 1-Isopropylacetylaceton) hergestellt und auf analoge Weise zu den obigen Beispielen 7 und 8 mit FOYE als En-Reduktase umgesetzt.

Die drei Oxime besitzen im Fall des unsubstituierten 3-Oximinoacetylacetons (3a) von Vergleichsbeispiel 1 zwei gleiche Enden CH₃- bzw. je ein Ende mit sperrigem Substituenten, nämlich Phenyl für α-Acetyl-α-oximinoacetophenon (3b) in Vergleichsbeispiel 2 bzw. Isobutyl für 1-Isopropyl-3-oximinoacetylaceton (3c) in Vergleichsbeispiel 3.

Während der Reaktionen wurden photometrische Aktivitätsmessungen durchgeführt, bei denen die Enzymaktivität anhand des Verbrauchs des Coenzyms NAD(P)H/H⁺ bestimmt wurde. Die Ergebnisse sind in nachstehender Tabelle 3 angeführt.

**Tabelle 3: Reaktivitätsscreening mit den Diketon-Oximen (3a) bis (3c)**

| **Vergleichsbeispiel** | **Oxim** | **Aktivität [U/mg]** |
|---|---|---|
| 1 | 3a | 6 |
| 2 | 3b | 18 |
| 3 | 3c | 23 |

Somit zeigte FOYE gegenüber allen drei Diketon-Oximen relativ hohe Aktivität. Nichtsdestotrotz konnte in keinem der drei Fälle mittels GC-FID das entsprechende Pyrazin-Produkt detektiert werden. Ohne sich auf eine Theorie festlegen zu wollen, nehmen die Erfinder an, dass der Grund die Gegenwart von zweier reaktiver Carbonylgruppen an den Ausgangsprodukten ist, so dass entweder reaktive Keton-Seitenketten am ringgeschlossenen Pyrazin gebildet werden, die mit einem weiteren enzymatisch reduzierten Oxim unter Ausbildung von Oligomeren reagieren können, oder einer solche Reaktion mit der zweiten Carbonylgruppe bereits am intermediär gebildeten Dimer, d.h. vor dem Ringschluss zum Pyrazin erfolgt. Tatsache ist, dass das erfindungsgemäße Verfahren offenkundig nur mit Oximen von Acetessigsäure-Derivaten, nicht aber mit Oximen von Acetylaceton- oder Acetylbenzophenon-Derivaten durchführbar ist.

### Beispiele 51 bis 54

Herstellung von Dimethylpyrazindicarbonsäureestern (2) in präparativem Maßstab unter Verwendung von lyophilisierten *E. coli-*Ganzzellen 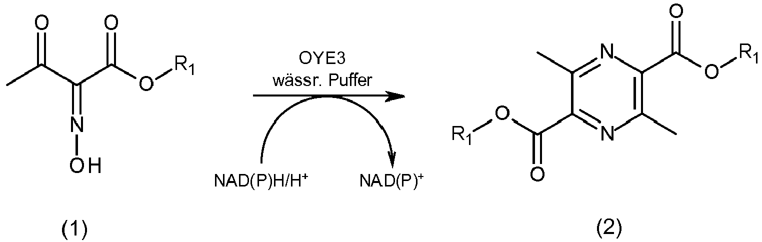
(1c) R₁ = Allyl
(1e) R₁ = Benzyl
(1f) R₁ = Heptyl
(1g) R₁ = 2-Methoxyethyl

Zur Untersuchung der erzelbaren Ausbeuten der Enzymreaktionen zu den gewünschten Pyrazindicarbonsäureestern (2) wurden die in den Synthesebeispielen 8 und 10 bis 12 hergestellten 2-Oximinoacetessigsäureester (1c), (1e), (1f) und (1g) wiederum in einer Konzentration von 10 mM mit 0,5 mM NAD(P)H/H⁺, 50 mM Glucose und 1 mg/ml GDH in 100 mM Tris-HCI-Puffer, pH 7,5, mit OYE3 als En-Reduktase umgesetzt, wobei in diesen Fällen jedoch ein Gesamtvolumen von 200 ml sowie lyophilisierte *E. coli*-Ganzzellen (1,5 g) als Enzympräparat eingesetzt wurden. Die Reinigung erfolgte in allen Fällen durch Extraktion mit Chloroform und anschließende Flash-Chromatographie.

### Beispiel 51: Herstellung von 3,6-Dimethylpyrazin-2,5-dicarbonsäurediallylester (2c)

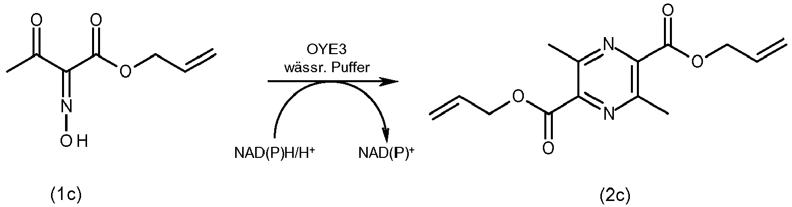

Die Reinigung mittels Flash-Chromatographie (EtOAc:Cyclohexan = 1:4, Rf = 0,39) ergab die Titelverbindung in Form eines gelblichen Feststoffs (Ausbeute: 21 % d. Th.).
Fp.: 58-59 °C
¹H-NMR (300 MHz, CDCl₃): δ = 6,06 (ddt, *J* = 16,3, 10,4, 5,9 Hz, 2H), 5,51-5,40 (m, 2H), 5,36-5,29 (m, 2H), 4,91 (dt, *J* = 5,9, 1,3 Hz, 4H), 2,80 (s, 6H). ¹³C-NMR (75 MHz, CDCl₃): δ = 164,7, 151,0, 143,9, 131,3, 119,7, 66,9, 22,4.

### Beispiel 52: Herstellung von 3,6-Dimethylpyrazin-2,5-dicarbonsäuredibenzylester (2e)

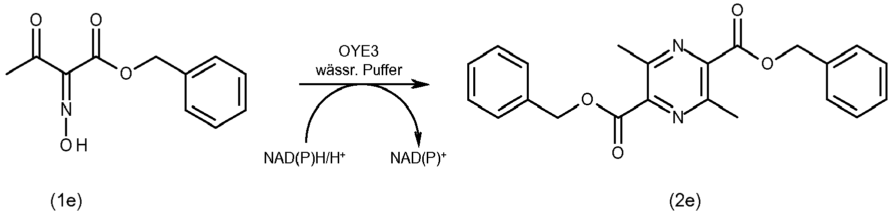

Die Reinigung mittels Flash-Chromatographie (EtOAc:Cyclohexan = 1:5, Rf = 0,29) ergab die Titelverbindung in Form eines farblosen Feststoffs (Ausbeute: 30 % d. Th.).
Fp.: 106-107 °C (Lit.: 108 °C; Laver et al., J. Chem. Soc. 0, 1474-1483 (1959))
¹H-NMR (300 MHz, CDCl₃): δ = 7,52-7,33 (m, 10H), 5,45 (s, 4H), 2,76 (s, 6H). ¹³C-NMR (75 MHz, CDCl₃): δ = 164,7, 151,0, 143,9, 135,1, 128,7, 128,6, 128,5, 67,9, 22,3.

### Beispiel 53: Herstellung von 3.6-Dimethylpyrazin-2,5-dicarbonsäurediheptylester (2f)

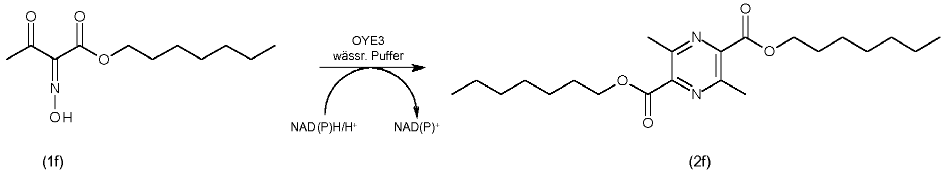

Die Reinigung mittels Flash-Chromatographie (EtOAc:Cyclohexan = 1:5, Rf = 0,33) ergab die Titelverbindung in Form eines gelblichen Feststoffs (Ausbeute: 30 % d. Th.).
Fp.: 55-56 °C
¹H-NMR (300 MHz, CDCl₃): δ = 4,41 (t, *J* = 6,9 Hz, 4H), 2,78 (s, 6H), 1,89-1,71 (m, 4H), 1,50-1,19 (m, 17H), 0,88 (dd, *J* = 8,8, 4,9 Hz, 6H). ¹³C-NMR (75 MHz, CDCl₃): δ = 164,7, 151,0, 143,9, 66,5, 31,7, 28,9, 28,5, 25,8, 22,5, 22,3, 14,0.

### Beispiel 54: Herstellung von 3,6-Dimethylpyrazin-2,5-dicarbonsäuredi(2-methoxyethyl)ester (2g)

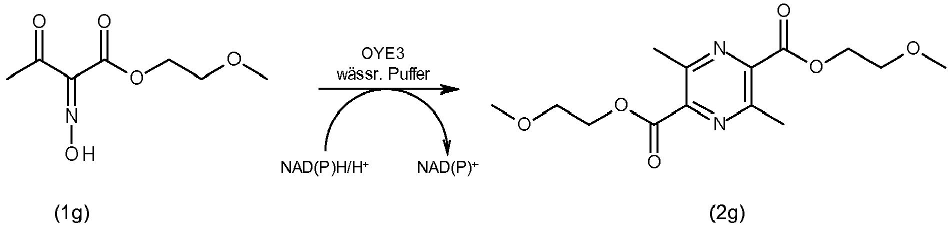

Die Reinigung mittels Flash-Chromatographie (EtOAc:Cyclohexan = 1:1, Rf = 0,30) ergab die Titelverbindung in Form eines farblosen Feststoffs (Ausbeute: 35 % d. Th.).
Fp.: 66-67 °C
¹H-NMR (300 MHz, CDCl₃): δ = 4,60-4,52 (m, 4H), 3,80-3,71 (m, 4H), 3,41 (s, 6H), 2,78 (s, 6H). ¹³C-NMR (75 MHz, CDCl₃): δ = 164,7, 151,0, 143,9, 70,1, 65,0, 59,0, 22,2.

Ohne sich auf eine Theorie festlegen zu wollen, nehmen die Erfinder an, dass der Grund für die relativ geringen Ausbeuten die Gegenwart von Esterasen im Ganzzell-Enzympräparat war, die möglicherweise die Ester zu den jeweiligen, nicht mit Chloroform aus der wässrigen Phase extrahierbaren Carbonsäuren hydrolysiert hatten.

Weitere Untersuchungen zur Aufklärung dieses Umstands sowie zur Optimierung der Reaktionsbedingungen sind derzeit Gegenstand der Forschungen der Erfinder.

Jedenfalls stellt die überraschende Entdeckung der Erfinder, dass En-Reduktasen in der Lage sind, Oxime zu reduzieren, einen neuen Mechanismus in der Biokatalyse dar, der die Grundlage für das erfindungsgemäße Verfahren ist. Durch dieses neue Verfahren der Erfindung können nun 3,6-disubstituierte Pyrazin-2,5-dicarbonsäure-Derivate auf deutlich reproduzierbarere Weise und mit praktisch 100%igem Umsatz aus entsprechenden Oximen hergestellt werden.

## Patentansprüche

1. Verfahren zur Herstellung von substituierten Pyrazinen ausgehend von aktivierten Ketoximen mittels enzymatischer Katalyse, wobei ein durch die nachstehende allgemeine Formel (1) dargestelltes Derivat der 2-Oximinoacetessigsäure in Gegenwart eines Enzyms aus der Klasse der En-Reduktasen (ER) und eines Hydridionen übertragenden Coenzyms wie NADH/H⁺ oder NADPH/H⁺ (NAD(P)H/H⁺) in wässriger Pufferlösung unter Dimerisierung, Ringschluss und Aromatisierung zum 3,6-disubstituierten Pyrazin-2,5-dicarbonsäure-Derivat (2) umgesetzt wird: 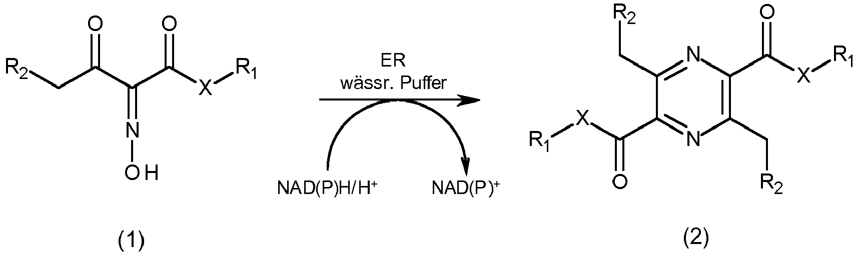 worin
X aus O, S, NH und NR₁ ausgewählt ist;
R₁ jeweils unabhängig voneinander aus linearen oder verzweigten, gesättigten oder ungesättigten aliphatischen und aromatischen Kohlenwasserstoffen mit 1 bis 10 Kohlenstoffatomen ausgewählt ist, in denen ein oder mehrere Kohlenstoffatome gegebenenfalls durch ein aus Sauerstoff, Stickstoff und Schwefel ausgewähltes Heteroatom ersetzt sind; und
R₂ aus Wasserstoff und den Optionen für R₁ ausgewählt ist.

2. Verfahren nach Anspruch 1, **dadurch gekennzeichnet, dass** R₁ aus C₁₋₆-Alkyl, C₁₋₆-Alkenyl, Phenyl und Benzyl ausgewählt ist, worin ein oder mehrere Kohlenstoffatome gegebenenfalls durch Sauerstoff ersetzt sind.

3. Verfahren nach Anspruch 1 oder 2, **dadurch gekennzeichnet, dass** R₂ aus Wasserstoff und C₁₋₆-Alkyl ausgewählt ist.

4. Verfahren nach Anspruch 3, **dadurch gekennzeichnet, dass** R₂ aus Wasserstoff und Methyl ausgewählt ist.

5. Verfahren nach einem der Ansprüche 1 bis 4, **dadurch gekennzeichnet, dass** X aus O und NH ausgewählt ist.

6. Verfahren nach einem der Ansprüche 1 bis 5, **dadurch gekennzeichnet, dass** die En-Reduktase aus der OYE-Familie (Old Yellow Enzymes) ausgewählt ist.

7. Verfahren nach Anspruch 6, **dadurch gekennzeichnet, dass** das Enzym aus den folgenden ausgewählt ist:
OYE1 von *Saccharomyces pastorianus,*
OYE2 von *Saccharomyces cerevisiae,*
OYE3 von *Saccharomyces cerevisiae,*
12-Oxophytodienoat-Reduktase 3 (OPR3) von *Arabidopsis thaliana,*
xenobiotischer Reduktase A (XenA) von *Pseudomonas putida* II-B und
En-Reduktase von *Ferrovum* sp. JA12 (FOYE).

8. Verfahren nach einem der Ansprüche 1 bis 7, **dadurch gekennzeichnet, dass** die wässrige Pufferlösung einen pH von 7 bis 8 aufweist.

9. Verfahren nach Anspruch 8, **dadurch gekennzeichnet, dass** die wässrige Pufferlösung einen pH von etwa 7,5 aufweist.

10. Verfahren nach einem der Ansprüche 1 bis 9, **dadurch gekennzeichnet, dass** die Reaktion bei einer Temperatur von 20 °C bis 35 °C durchgeführt wird.

11. Verfahren nach Anspruch 10, **dadurch gekennzeichnet, dass** die Reaktion bei einer Temperatur von 30 °C durchgeführt wird.

12. Verfahren nach einem der Ansprüche 1 bis 11, **dadurch gekennzeichnet, dass** das Coenzym unter Verwendung einer Dehydrogenase rezykliert wird.

13. Verfahren nach Anspruch 12, **dadurch gekennzeichnet, dass** zur Rezyklierung des Coenzyms eine Kombination aus Glucose und Glucose-Dehydrogenase (GDH) oder aus Phosphit und Phosphit-Dehydrogenase (PDH) eingesetzt wird.

14. Verfahren nach Anspruch 13, **dadurch gekennzeichnet, dass** zur Rezyklierung des Coenzyms eine Kombination aus Glucose und Glucose-Dehydrogenase (GDH) eingesetzt wird.
